# EUROPEAN PATENT SPECIFICATION

(11) **EP 4 009 869 B1**
(45) Date of publication and mention of the grant of the patent: **28.01.2026**
(21) Application number: 20849230.6
(22) Date of filing: 05.08.2020
(51) Int. Cl.: A61B 5/15, A61B 5/154

(54) **BLOOD COLLECTION DEVICE THAT SEQUESTERS AN INITIAL COLLECTED PORTION**
BLUTENTNAHMEVORRICHTUNG MIT SEQUESTRIERUNG EINES ANFÄNGLICHEN GESAMMELTEN TEILS
DISPOSITIF DE COLLECTE DE SANG QUI EMPRISONNE UNE PARTIE RECUEILLIE INITIALE

(30) Priority: 07.08.2019 US 201962883941 P
(43) Date of publication of application: 15.06.2022
(73) Proprietor: Becton, Dickinson and Company, Franklin Lakes, NJ 07417 (US)
(72) Inventor: ARMSTRONG, Robert Edward, Hunt Valley, MD 21030 (US); CHAN, Wai Ting, Cambridgeshire GB CB24 3EG (GB); DEANE, Steven Charles, Cambridge GB CB23 8EG (GB); WHITTOME, Samuel Edmund, Cherry Hinton GB CB1 9GQ (GB); PARKER, Thomas Edward, Cambridge GB CB23 8SR (GB); SARRIAS, Merissa Lim, Hauxton GB CB22 5HS (GB); WILLIAMS, Erik David, Cambridge GB CB2 9LU (GB)
(74) Representative: dompatent
(86) International application number: PCT/US2020/044993
(87) International publication number: WO 2021/026223

(56) References cited:
- WO-A1-2016/201406
- US-A- 4 212 308
- US-A- 4 212 308
- US-A- 5 147 329
- US-A1- 2014 309 553
- US-A1- 2017 020 427
- US-A1- 2017 027 491
- US-A1- 2018 353 117
- US-A1- 2019 076 074
- US-A1- 2021 361 207
- US-A1- 2022 304 601

## Description

### CROSS-REFERENCE TO RELATED APPLICATIONS

This application claims the benefit of priority from U.S. Provisional Application No. 62/883,941 filed August 7, 2019.

### TECHNICAL FIELD

The present technology relates to a device for trapping an initial flow of blood during a blood collection process. The present invention relates to a diversion device according to the preamble of claim 1. Such a diversion device is known from US 2018/353117 A1.

### BACKGROUND

A blood culture test is presently the preferred method for identifying bacteremia and septicemia (sepsis). Sepsis is a body-wide response to a bacterial infection of the blood stream that can cause organ failure and death. Sepsis kills every one in six infected patients. Moreover, half of all in-hospital deaths involve sepsis. In fact, sepsis kills more people than AIDS, breast cancer and prostate cancer combined. Sepsis affects more hospital patients than any other diagnosis.

Unfortunately, the United States healthcare system spends over $4 billion each year on unnecessary treatment associated with false positive blood culture results. *See* Oren Zwang & Richard K. Albert, Analysis of Strategies to Improve Cost Effectiveness of Blood Cultures, 1 J. Hosp. Med. 272 (Sep. 2006). Moreover, "[i]t is currently accepted that most organisms identified as contaminants in blood cultures originate from the skin of the patient." Robert A. Garcia et al., Multidisciplinary Team Review of Best Practices for Collection and Handling of Blood Cultures to Determine Effective Interventions for Increasing the Yield of True -Positive Bacteremia, Reducing Contamination, and Eliminating False-Positive Central Line-Associated Bloodstream Infections, 43 Am. J. Infect. Control 1222 (Nov. 2015).

Devices that help manage the flow of blood during the collection process to reduce infection or contamination have been previous described in the prior art. For example, one device described in US4212308A to Edward P. Percarpio, which was filed on December 27, 1977, uses multiple one-way valves in parallel to prevent backflow of blood towards the patient without significantly decreasing the blood flow during the collection process. However, such devices do not aid in eliminating the potential for contamination of blood from microbial skin flora. Thus, during a blood collection process, there is a need for a device capable of diverting and trapping an initial flow of blood from a patient that might contain contaminants from the skin of that patient in order to reduce the number of false positives. One such device is described in WO2019018324 to Milan Ivosevic, which was filed on July 17, 2018 as PCT/US2018/042367. Another device is described in US/2018/353117A1 to Gregory J. Bullington et al., which was filed on June 7, 2017 as PCT/US67/517681. While this device is designed to reduce sample contamination during the collection process, the device described uses hydrophilic material to absorb and sequester an initial blood sample. There remains a need for a simpler device that can effectively reduce contamination of samples during the collection process.

### BRIEF SUMMARY

According to the present invention there is provided a diversion device according to claim 1. Preferred embodiments of the present invention are defined in the dependent claims. A diversion device that traps an initial flow of blood in a diversion chamber of the diversion device as recited in the appended claims is described herein. The diversion chamber may be defined, in part, by a flow path defined by a channel or series of channels that terminate in a diversion chamber valve. The diversion chamber valve is a passage configured to permit the flow of air therethrough but that does not permit a liquid such as the collected blood to flow through it. After the diversion chamber is filled, the collected blood begins to flow through a collected sample valve and is drawn through a bypass flow chamber of the diversion device and into a collection vessel in fluid communication with and downstream from the diversion device.

One aspect of the present disclosure relates to a diversion device comprising: (1) a housing having an inlet conduit and an outlet conduit, wherein the housing is configured to receive an initial flow of blood and a subsequent flow of blood through the inlet conduit, and wherein the housing is configured to allow the subsequent flow of blood to exit the diversion device through the outlet conduit; (2) a diversion chamber defined by a flow path defined by a channel or series of channels that terminate in a diversion chamber valve; (3) a collected sample valve; and (4) a bypass flow chamber, wherein the collected sample valve is configured to permit the subsequent flow of fluid to enter the bypass flow chamber, and the bypass flow chamber is configured to permit the subsequent flow of fluid to exit the diversion device through the outlet conduit.

Both the diversion chamber valve and the collected sample valve deploy hydrophobic material and smaller diameter passages or channel to increase flow resistance to the flow of blood through the channel. Non-limiting examples of hydrophobic materials include, e.g., polytetrafluoroethylene (PTFE), polypropylene, or other conventional non-polar polymers. Suitable polymers will have sufficient thermal stability so that the device can be sterilized.

The diversion chamber valve is configured to completely prevent the flow of liquid through the valve. When the diversion chamber is filling with the initial portion of blood, the collected sample valve is configured to provide a flow resistance that will not pass the initial portion of blood collected into a collection vessel. When the diversion chamber is full, the flow resistance of the diversion chamber valve is such that the subsequent portion of blood flowing into the diversion device will "break through" the flow resistance of the collected sample valve and flow into the bypass flow chamber and through the outlet conduit.

A portion of the housing comprises a hydrophilic material, such as carboxymethylcellulose ("CMC"). Hydrophilic materials are optionally used to enhance the motive force for the fluid by, for example, wicking the liquid to drive it through the device.

In some embodiments, a cross-sectional area of the diversion chamber is larger than a cross-sectional area of the bypass flow chamber. In some embodiments, the bypass flow chamber comprises a tube. In some embodiments, the housing comprises a housing shell, wherein the housing shell contains the inlet conduit on one end and the outlet conduit on the opposite end. In some embodiments, a vacuum pressure created by a collection vessel coupled to the diversion device draws the initial flow of blood into the diversion chamber. The bypass flow chamber is configured to permit the subsequent flow of fluid to exit the diversion device using only the vacuum pressure created by the collection vessel coupled to the diversion device.

Another aspect of the present disclosure relates to a blood collection kit comprising: instructions to assemble a blood collection pathway from a patient to a collection vessel, wherein the blood collection pathway comprises a first needle piercing the skin of the patient and a diversion device, and wherein the collection vessel has a sub-atmospheric internal pressure that draws (a) an initial flow of blood from the patient through the first needle and into the diversion device and (b) a subsequent flow of blood through the first needle and the diversion device, respectively, and into the collection vessel, and wherein the blood collection pathway is a closed system that prevents an initial flow of air through the diversion device from being vented into the atmosphere.

In some embodiments, the blood collection pathway further comprises a holder having a second needle piercing a cap of the collection vessel. In some embodiments, the diversion device is integrated with the holder. In some embodiments, the diversion device and the holder are separate units. In some embodiments, the diversion device is integrated with a first needle used to pierce a vein or an artery of the patient. In some embodiments, the diversion device and the first needle are separate units that are in close proximity to each other, or in some embodiments, right next to each other. In some embodiments, the collection vessel contains one or more bacterial growth media, an antibiotic scavenger, or a pH sensor.

Yet another aspect of the present disclosure relates to a blood collection method, which is not according to the present invention, comprising: assembling a blood collection pathway from a patient to a collection vessel, wherein the blood collection pathway comprises a first needle piercing the skin of the patient and a diversion device, and wherein the collection vessel has a sub-atmospheric internal pressure that draws (a) an initial flow of blood from the patient through the first needle and into the diversion device and (b) a subsequent flow of blood through the first needle and the diversion device, respectively, and into the collection vessel, and wherein the diversion device comprises: (1) a housing having an inlet conduit and an outlet conduit, wherein the housing is configured to receive an initial flow of blood and a subsequent flow of blood through the inlet conduit, and wherein the housing is configured to allow the subsequent flow of blood to exit the diversion device through the outlet conduit; (2) a diversion chamber defined by a flow path defined by a channel or series of channels that terminate in a diversion chamber valve; (3) a collected sample valve; and (4) a bypass flow chamber, wherein the collected sample valve is configured to permit the subsequent flow of fluid to enter the bypass flow chamber, and the bypass flow chamber is configured to permit the subsequent flow of fluid to exit the diversion device through the outlet conduit.

The blood collection pathway is a closed system that prevents an initial flow of air through the diversion device from being vented into the atmosphere. In some embodiments, the blood collection pathway further comprises a holder having a second needle piercing a cap of the collection vessel. In some embodiments, the diversion device is integrated with the holder. In some embodiments, the diversion device and the holder are separate units. In some embodiments, the diversion device is integrated with a first needle used to pierce a vein or an artery of the patient. In some embodiments, the diversion device and the first needle are separate units that are in close proximity to each other, or in some embodiments, right next to each other.

A diversion device for collecting a biological sample is described herein. The diversion device has an inlet for receiving a biological sample collected from a patient. The diversion device has an outlet for delivering the collected biological sample to a collection vessel, the collection vessel being under sub-atmospheric pressure. The diversion device also has a first channel into which a first portion of the collected biological sample flows upon commencement of sample collection. The first channel has a first valve such that air in the first channel exits the first channel through the valve as the collected sample fills the first channel. The device also has a second channel into which a second portion of the collected sample flows after the first channel is substantially filled with collected sample. The second channel is in fluid communication with the first channel through a second valve. The diversion device outlet is adapted for attachment to a needle with a lumen. The needle is adapted to pierce a seal on the collection vessel, such that the sub-atmospheric pressure of the collection vessel draws the biological sample from the device to the collection vessel.

Optionally, the inlet of the diversion device is adapted for connection to a line set for collecting a biological sample from a patient. Typically, line sets have a sample collection needle and a collection tube. Optionally the sample collection needle is a butterfly needle selected from the group consisting of a single-winged butterfly needle or a dual wing butterfly needle. Optionally, the diversion device is integrated into a wing of the butterfly wing needle.

Optionally the diversion device is adapted to be coupled to an adapter that fluidically couples to a collection vessel. The collection vessel is sealed and has an internal pressure that is less than atmospheric pressure. The adapter is coupled to the diversion device by any conventional coupling (e.g. threaded connection, snap connection, luer connector, etc.

The valves of the diversion device operate as follows. The first valve operates to let air escape from the first channel but retains the sample collected in the first channel. The air that passes from the first channel is received from the second channel is drawn out of the diversion device by the reduced pressure in the collection vessel. The second valve operates such the sample does not flow from the first channel to the second channel until the time when the first channel is filled with sample, which overcomes for liquid flow resistance of the second valve. Both valves are hydrophobic flow restrictors. Both valves have a barrier with one or more openings the provided liquid flow resistance. Optionally, the flow restrictors are barriers that have an orifice of about 2mm or less therein. Optionally, the flow restrictors have multiple barriers with one or more orifices in each barrier. Optionally, the diversion device has an orifice with a diameter of about 0.5 µm or less.

The first channel in the diversion device can be a serpentine channel or a straight channel. Optionally, the first channel has diameter of about 3 to about 4 mm.

Optionally, the first and second valves are hydrophobic membranes. Such membranes are porous and the size of the pores is about 0.45 µm or less. Examples of hydrophobic materials from which the hydrophobic flow restrictors or membranes are made include polytetrafluoroethylene (PTFE) or polypropylene.

Described herein is a diversion device assembly for collecting a biological sample. The assembly includes a butterfly needle and a diversion device integrated on the butterfly needle. the diversion device has an inlet for receiving a biological sample collected from a patient. The diversion device has an outlet for delivering the collected biological sample to a collection vessel. The collection vessel is typically under sub-atmospheric pressure (i.e. the inside of the container has a pressure that is less than atmospheric pressure. The diversion device has a first channel into which a first portion of the collected biological sample flows upon commencement of sample collection. The first channel has a first valve such that air in the first channel exits the first channel through the valve as the collected sample fills the first channel. The valve is in fluid communication with the outlet of the diversion device so that any air exiting the first channel is drawn from the device into the collection vessel. That is, the diversion device is not vented to the atmosphere. The diversion device has a second channel into which a second portion of the collected sample flows after the first channel is substantially filled with collected sample, the second channel is in fluid communication with the first channel through a second valve. The second channel is also in fluid communication with an adapter wherein the adaptor receives the collected biological sample from the second channel and wherein the adapter outlet is adapted for attachment to a needle with a lumen, the needle adapted to pierce a seal on a collection vessel, such that the sub-atmospheric pressure of the collection vessel draws the biological sample from the diversion device into the collection vessel. The first and second valves are as described previously.

### BRIEF DESCRIPTION OF DRAWINGS

FIG. 1 illustrates a blood collection system comprising a diversion device in accordance with the present technology.
FIG. 2 illustrates an embodiment of a diversion device that is integrated with a holder in accordance with the present technology.
FIG. 3 illustrates an embodiment of a diversion device in accordance with the present technology.
FIGS. 4A-4C illustrate alternate embodiments of a diversion device in accordance with the present technology.
FIG. 5 is a schematic illustrating the path of flow of blood through a diversion device in accordance with the present technology into a blood collection bottle.
FIGS. 6A-6C illustrate the sequence of blood flow into one embodiment of a diversion device in accordance with the present technology, first filling the diversion chamber before flowing through the bypass chamber.
FIG. 7 is an enlarged view of a diversion chamber valve of one embodiment of a diversion device in accordance with the present technology.
FIG. 8 is a plot of percent contamination left in a needle and/or needle with tubing as a function of volume of blood dispensed.

### DETAILED DESCRIPTION

Embodiments of the present disclosure are described in detail with reference to the drawing figures wherein like reference numerals identify similar or identical elements. It is to be understood that the disclosed embodiments are merely examples of the disclosure, which may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

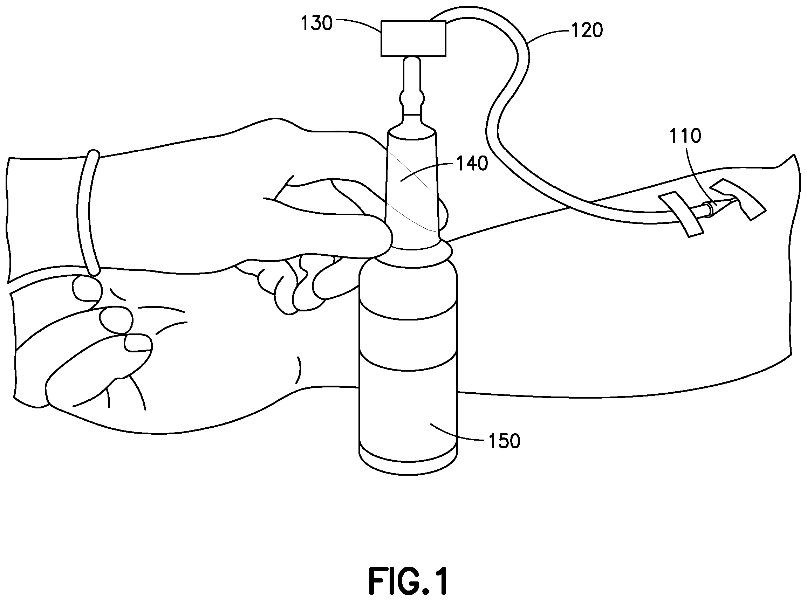

FIG. 1 illustrates a blood collection system comprising a diversion device in accordance with the present technology. As shown in FIG. 1, the blood collection system includes First Needle 110, Tubing 120, Diversion Device 130, Holder 140, and Collection Bottle 150. During the process of collecting a blood sample from a patient, First Needle 110 is used to pierce a vein or an artery of the patient. Driven by the vacuum pressure created by Collection Bottle 150, and patient blood pressure, blood from the patient is directed toward Collection Bottle 150 through Tubing 120. An initial flow of blood passes through Tubing 120 and is trapped in a diversion chamber within Diversion Device 130. A subsequent flow of blood is collected in Collection Bottle 150. Along the way, the subsequent flow of blood passes by the diversion chamber of the Diversion Device 130 to a second needle of Holder 140.

In some embodiments, the blood collection system of FIG. 1 may be implemented using one of Becton, Dickinson and Company's ("BD's") Vacutainer^{®} blood collection sets, such as BD's Vacutainer^{®} push button blood collection set, BD's Vacutainer^{®} Safety-Lok^{™} blood collection set, or BD's Vacutainer^{®} UltraTouch^{™} push button blood collection set. Therefore, in some embodiments, an adapter may be implemented using BD's Vacutainer^{®} Multiple Sample Luer Adapter. Moreover, in some embodiments, Holder 140 may be implemented using BD's Vacutainer^{®} One Use Holder.

As shown in FIG. 1, Diversion Device 130 is a separate unit that is in proximity to Holder 140. However, in other embodiments, Diversion Device 130 may be integrated with Holder 140. In some embodiments, the Diversion Device 130 is integrated with the First Needle 110 used to pierce a vein or an artery of the patient. In some embodiments, the Diversion Device 130 and the First Needle 110 are separate units that are in close proximity to each other, or in some embodiments, right next to each other. Moreover, the size of Diversion Device 130 may be changed to adjust the amount of blood that is initially directed into the diversion chamber within Diversion Device 130. The volume of blood diverted into the diversion device may also be changed depending on the proximity of the diversion device to the first needle. For example, in some embodiments, if the diversion device is immediately behind the first needle (e.g., as part of the winged butterfly first needle), the diversion device may be configured to direct less than about 150 µL of blood into its diversion chamber. In some embodiments, the diversion device may be configured to direct less than about 30-50 µL of blood into its diversion chamber.

In some embodiments, Diversion Device 130 may include an indicator for providing feedback relating to the amount of collected blood. For example, Diversion Device 130 may include a flow meter that indicates how much blood has been collected inside Collection Bottle 150. The flow meter could minimize potentially false negative blood cultures by helping to ensure that health care workers collect an adequate amount of blood. Furthermore, in some embodiments, a transmitter may be communicatively coupled to the indicator for wirelessly transmitting information relating to the amount of collected blood to a receiver. In such embodiments, the receiver may be communicatively coupled to a display device configured to display information relating to the amount of collected blood.

Collection Bottle 150 may be constructed of glass, plastic, or other suitable materials. In some embodiments, Collection Bottle 150 may be implemented using one of BD's BACTEC^{™} culture vials or one of BD's Vacutainer^{®} blood collection tubes. In some embodiments, Collection Bottle 150 may contain liquids and/or solid additives, such as a bacterial growth media, an antibiotic scavenger, or a pH sensor. In some embodiments, Collection Bottle 150 may contain one of BD's blood culture medias, such as BD's BACTEC^{™} Peds Plus^{™} medium, BD's BACTEC^{™} Plus Aerobic medium, BD's BACTEC^{™} Plus Anaerobic medium, BD's BACTEC^{™} Lytic Anaerobic medium, BD's BACTEC^{™} Standard Aerobic medium, or BD's BACTEC^{™} Standard Anaerobic medium.

As mentioned above, most organisms identified as contaminants in blood cultures originate from the skin of the patient. These contaminants are typically introduced into a patient's blood sample by the venipuncture and the initial flow of blood from the patient into a collection bottle. In the blood collection system of FIG. 1, the initial flow of blood is diverted and trapped in the diversion chamber of Diversion Device 130. As a result, the blood collection system of FIG. 1, provides a means for potentially reducing the number of false positive blood cultures. Moreover, the inclusion of Diversion Device 130 in the blood collection system of FIG. 1, does not introduce additional workflow steps for health care workers relative to presently conventional techniques for collecting blood samples. For example, health care workers do not need to wait for a conduit or a chamber to partially or completely fill before inserting Collection Bottle 150 into Holder 140.

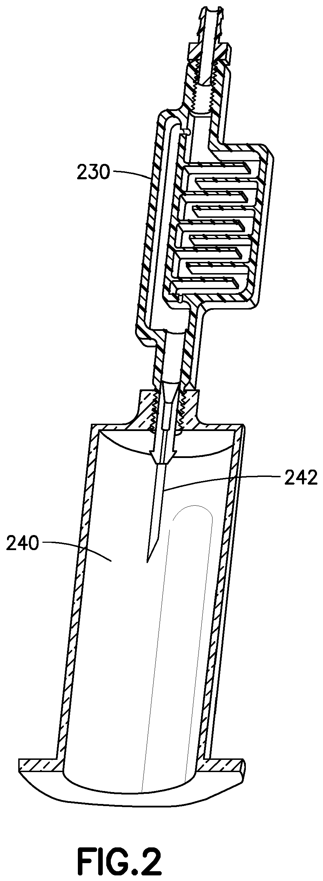

FIG. 2 illustrates an embodiment of a diversion device that is integrated with a holder in accordance with the present technology. As shown in FIG. 2, Diversion Device 230 is integrated with Holder 240, which includes a Second Needle 242. The Holder 240 is adapted to be received onto a bottle or collection device (not shown). Second Needle 242 provides a fluid channel from the Diversion Device 230 into the collection device. In those embodiments where Holder 240 is sealed prior to assembly with the Diversion Device 230, Second Needle 242 pierces through Holder 240 when the Holder 240 and Diversion Device are assembled together.

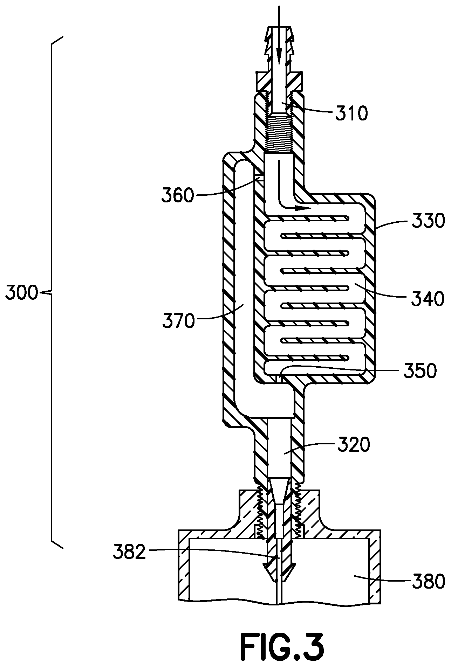

FIG. 3 illustrates an embodiment of a diversion device in accordance with the present technology. As shown in FIG. 3, Diversion Device 300 is connected to Holder 380, which includes Second Needle 382 that is pierced through a septum or cap with a septum port of a collection bottle.

As shown in FIG. 3, the Diversion Device 300 comprises a housing having an Inlet Conduit 310 and an Outlet Conduit 320, a Diversion Chamber 330 comprising a Channel or Series of Channels 340, a Diversion Chamber Valve 350, a Collected Sample Valve 360, and a Bypass Flow Chamber 370.

As noted with respect to FIG. 1, during the process of collecting a blood sample from a patient, a first needle is used to pierce a vein or an artery of the patient. Driven by the vacuum pressure created by the collection bottle, blood from the patient is directed toward collection bottle through tubing and the diversion device described herein. Referring to FIG. 3, an initial flow of blood passes through Inlet Conduit 310 and is trapped in Diversion Chamber 330 within Diversion Device 300. A subsequent flow of blood passes through the Bypass Flow Chamber 370 and is collected in the collection bottle. Along the way, the subsequent flow of blood passes from the Diversion Device 300 to Second Needle 382 of Holder 380.

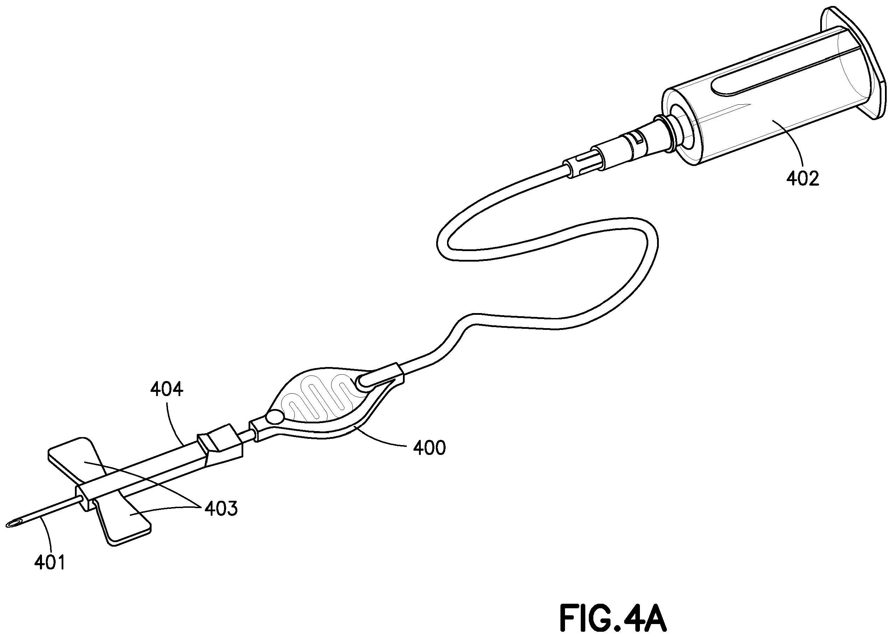

Collectively, FIGS. 4A-4C illustrate alternate embodiments of a diversion device in accordance with the present technology. As shown, the size and shape of the diversion device can be varied as well as its location in the blood collection system.

FIG. 4A illustrates an embodiment where Diversion Device 400 is in close proximity to, and in some embodiments, right next to a First Needle 401 that is used to collect blood from a patient. In contrast to the embodiment shown in FIG. 2, the Diversion Device 400 in the embodiment of FIG. 4A is not in close proximity to or right next to Holder 402 that is connected to a collection bottle. In this embodiment, the Diversion Device is in the line set and in close proximity to the sample collection site. As explained elsewhere herein, moving the Diversion Device closer to the collection site reduces the volume of blood that needs to be sequestered in the diversion chamber.

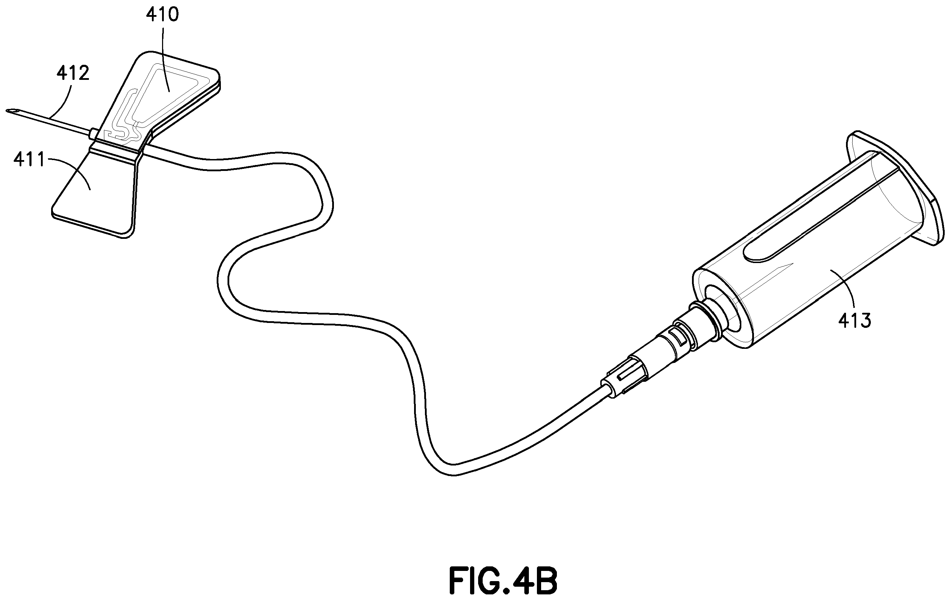

FIG. 4B illustrates an embodiment where Diversion Device 410 is part of a wing of a Dual-winged Butterfly First Needle 412 that is used to collect blood from a patient. As shown, the other Wing 411 of the Dual-winged Butterfly First Needle 412 does not contain a diversion device. In contrast to the embodiment shown in FIG. 2, the Diversion Device 410 in the embodiment of FIG. 4B is not in close proximity to or right next to Holder 413 that is connected to a collection bottle, but is in close proximity to the site at which the blood is collected from the patient.

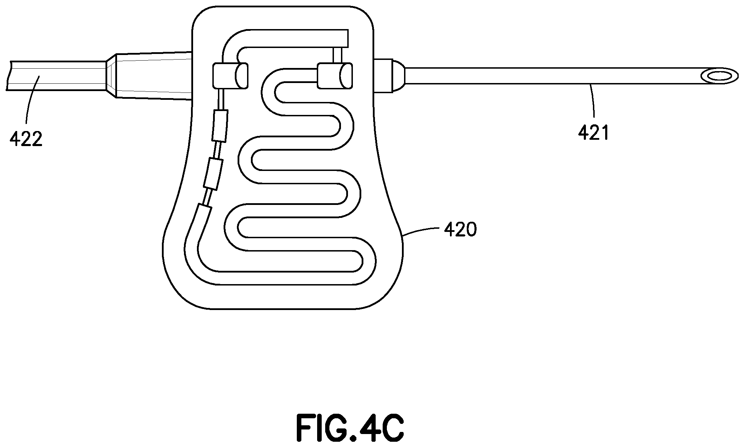

FIG. 4C illustrates a preferred embodiment where Diversion Device 420 is part of the wing of a Single-winged Butterfly First Needle 421 that is used to collect blood from a patient. In contrast to the embodiment shown in FIG. 2, the Diversion Device 420 in the embodiment of FIG. 4C is not in close proximity to or right next to holder that is connected to a collection bottle, but is in close proximity to the site at which the blood is collected from the patient. The collected blood flows into the Diversion Device 420 (first filling the diversion chamber) and then into Tubing 422 that is connected to a holder attached to a collection bottle.

As shown in FIGS. 4A-4C, First Needle 401, 412, and 421 may include one or more wings. For example, in FIG. 4A, First Needle 401 is a dual-winged butterfly needle with Wings 403. Wings can make it easier for a health care worker to grasp the first needle. However, in other embodiments of the present invention, wings can be omitted. In some embodiments, wings may be constructed of a flexible plastic material. In some embodiments, the first needle may also include a body. For example, in FIG. 4A, First Needle 401 is a dual-winged butterfly needle with Body 404. Body 404 may provide a health care worker with an indication that the vein or artery of a patient has been successfully pierced. For example, the Body 404 may be constructed of a translucent plastic material that allows a health care worker to see an initial flash of blood from a patient. In other embodiments, the body may be constructed of a transparent material or include a window. In some embodiments, the blood collection system of FIGS. 4A-4C may be implemented, in part, by using BD's Vacutainer^{®} push button blood collection set in combination with one of BD's BACTEC^{™} culture vials.

In some embodiments, the housing for the diversion device and/or a holder may be constructed of a plastic material, such as Acrylonitrile Butadiene Styrene ("ABS"). In some embodiments, tube may be constructed of a hydrophobic material. For example, in some embodiments, tube may be constructed of a plastic material, such as Polyethylene. In some embodiments, housing shell may be attached to a housing base by an ultrasonic welding process.

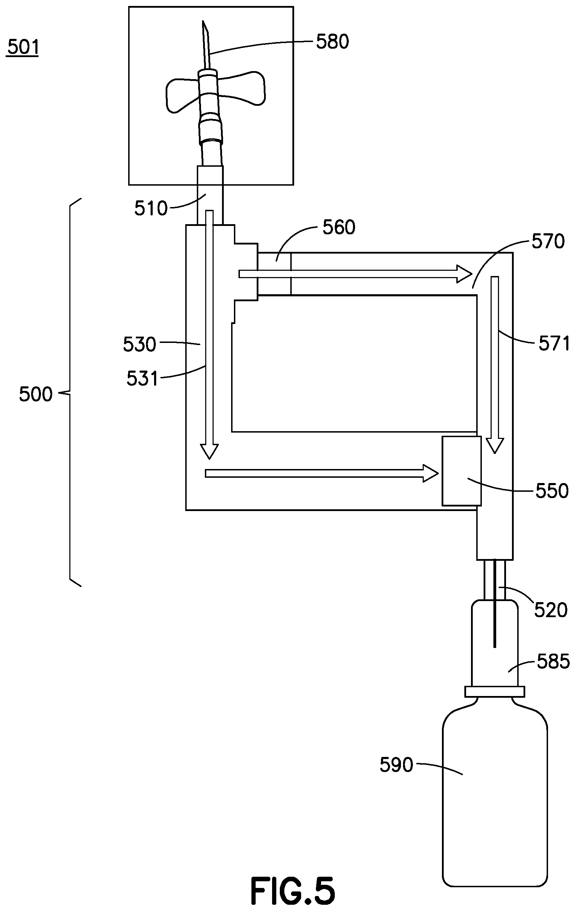

FIG. 5 is a schematic illustrating how initial and subsequent flows of blood from a patient may flow through a diversion device in accordance with the present technology. When Diversion Device 500 is used as part of a blood collection system 501, blood from a patient flows instantaneously under venous pressure through First Needle 580 located at the proximal end of the system 501. Driven by the vacuum pressure created by Collection Bottle 590 on the distal end of the system 501, blood from the patient flows into the Diversion Device 500 through Inlet Conduit 510 and, after the initial collected portion is diverted, the collected blood flows into the Collection Bottle 590. Collected Sample Valve 560 is illustrated as perpendicular to the path of incoming blood flow and Diversion Chamber 530. Preferably, Collected Sample Valve 560 of the Diversion Device 500 is as close to the First Needle 580 as possible with no stagnant flow regions (*see, e.g.,* FIG. 4C), which minimizes the volume of blood to be sequestered prior to permitting the blood to flow from the Diversion Chamber 530 and into the Collection Bottle 590 that is assemble to Holder 585. The path from the First Needle 580 into Diversion Chamber 530 should be as straight as possible, so that blood momentum is not impeded. The Collected Sample Valve 560 has a position (i.e., perpendicular to incoming blood) and a structure (i.e., a small orifice or hole in a hydrophobic material) that causes the initial portion of blood flowing into the Diversion Device 500 to preferentially flow into and fill the Diversion Chamber 530. Only after the Diversion Chamber 530 is filled is there sufficient force from the backed-up blood flow to overcome the flow resistance of the Collected Sample Valve 560, after which blood flows through the Collected Sample Valve 560. Non-limiting examples of hydrophobic materials used to construct the Collected Sample Valve 560 include, e.g., Polytetrafluoroethylene (PTFE) or polypropylene. In some embodiments, the small orifice or hole in the hydrophobic material of the Collected Sample Valve 560 has a diameter of about 0.2 mm. In alternate embodiments, Collected Sample Valve 560 is a membrane with multiple pores or holes. In some embodiments, each pore or hole of the membrane of Collected Sample Valve 560 has a diameter of about 5 µm. In alternate embodiments, each pore or hole of the membrane of Collected Sample Valve 560 has a diameter of about 0.45 µm. In some embodiments, the membrane of Collected Sample Valve 560 is made of a hydrophobic material. Non-limiting examples of hydrophobic materials used to construct the membrane of Collected Sample Valve 560 include, e.g., Polytetrafluoroethylene (PTFE) or polypropylene.

An initial flow of blood bypasses Collected Sample Valve 560 and flows into Diversion Chamber 530. Such a path reflects the path of least flow resistance for the blood, since, as noted above, flow through the Collected Sample Valve 560 requires that the flow resistance of the Collected Sample Valve 560 be overcome. Thus, the flow of the initial portion of blood into the Diversion Chamber 530 is the preferred flow path for the initial portion of the collected blood sample flowing into the Diversion Device 500.

Diversion Chamber 530 has a channel or series of channels that terminate in Diversion Chamber Valve 550. In some embodiments, the channel or series of channels of Diversion Chamber 530 have a diameter of about 3 to about 4 mm. In some embodiments, the length of the path through Diversion Chamber 530 is minimized to prevent unnecessary airflow restriction. In some embodiments, Diversion Chamber Valve 550 is of such a diameter that it is able to hold back the momentum of a column of liquid, thereby preventing any of the blood from passing through the Diversion Chamber Valve 550 and into the Bypass Flow Chamber 570. In some embodiments, Diversion Chamber Valve 550 has a diameter that is much smaller than about 0.2 mm. In alternate embodiments, Diversion Chamber Valve 550 is a membrane with multiple pores or holes. In some embodiments, each pore or hole of the membrane of Diversion Chamber Valve 550 has a diameter of about 5 µm. In alternate embodiments, each pore or hole of the membrane of Diversion Chamber Valve 550 has a diameter of about 0.45 µm. In some embodiments, the membrane of Diversion Chamber Valve 550 is made of a hydrophobic material. Non-limiting examples of hydrophobic materials used to construct the membrane of Diversion Chamber Valve 550 include, e.g., Polytetrafluoroethylene (PTFE) or polypropylene. In some embodiments, Diversion Chamber Valve 550 holds back much more static pressure than Collected Sample Valve 560.

FIG. 5 illustrates how an initial flow of Blood 531 from a patient may flow into Diversion Chamber 530. The initial flow of Blood 531 may contain contaminant bacteria (i.e. bacteria from the skin surface and not from the collected sample). As Diversion Chamber 530 begins to fill with initial flow of Blood 531, Diversion Chamber Valve 550 prevents the blood from flowing into the Outlet Conduit 520. However, if vacuum is applied, for example, through the vacutainer adapter, Diversion Chamber Valve 550 allows gas or air to flow through, but the collected blood cannot flow past Diversion Chamber Valve 550. The diversion Chamber Valve 550 is constructed of a hydrophobic material that allows air to pass through it, but not blood. Non-limiting examples of materials used to construct Collected Sample Valve 560 include, e.g., Polytetrafluoroethylene (PTFE) or polypropylene. The air that precedes the initial portion of blood into the Diversion Device travels though Outlet Conduit 520 through either valve. As such, Diversion Device 500 is a closed system. The initial flow of air through Diversion Device 500 is not vented to the atmosphere. Therefore, a health care worker does not need to wait for the air to be purged from Diversion Device 500 before connecting it to Collection Bottle 590. As a result, the initial flow of Blood 531 pushes air from Diversion Chamber 530 into Collection Bottle 590 through Diversion Chamber Valve 550. The portion of the initial flow of Blood 531 that fills the Diversion Chamber 530 becomes locked in place. Advantageously, this portion of the initial flow of Blood 531 likely contains the most contaminants (e.g., bacteria). As Diversion Chamber is filled with the initial flow of Blood 531, it closes off the flow of blood therethrough.

FIG. 5 also illustrates how a subsequent flow of Blood 571 from the patient may flow through Inlet Conduit 510 towards Collection Bottle 590. Once the Diversion Chamber 530 is filled up, the pressure at Collected Sample Valve 560 starts to build and permits subsequent flow of Blood 571 through into a Bypass Flow Chamber 570. The subsequent flow of Blood 571 passes through Bypass Flow Chamber 570 and exits the Diversion Device 500 through Outlet Conduit 520 into Collection Bottle 590.

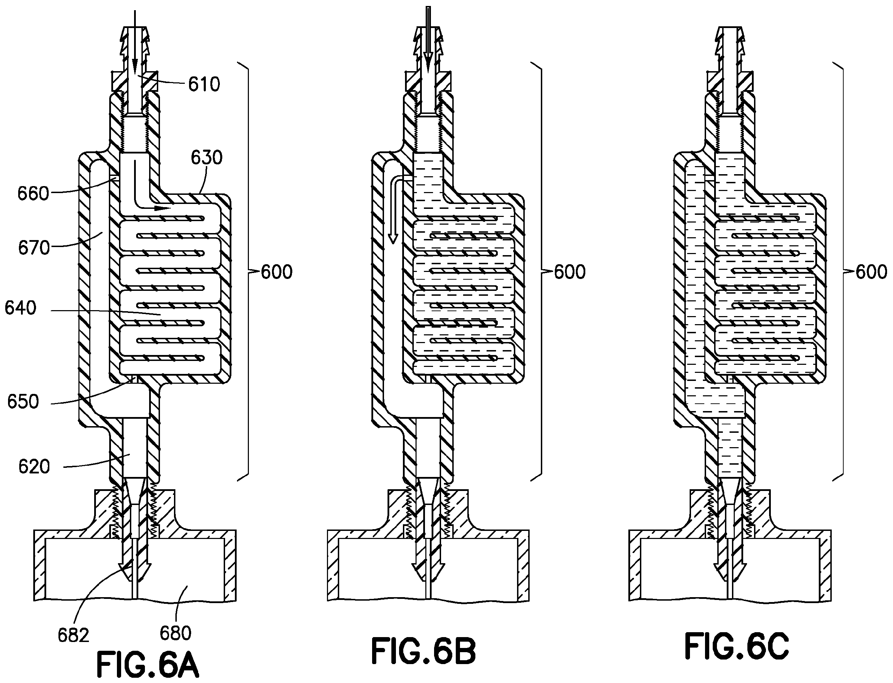

FIGS. 6A-6C illustrate the sequence of blood flow into one embodiment of a diversion device in accordance with the present technology, first filling the diversion chamber before flowing through the bypass chamber. In this embodiment, Diversion Device 600 is attached to Holder 680, which is a vacutainer adapter as illustrated. The labels shown in FIG. 6A apply equally to FIG. 6B and FIG. 6C. As shown in FIGS. 6A-6C, the Diversion Device 600 comprises a housing having an Inlet Conduit 610 and an Outlet Conduit 620, a Diversion Chamber 630 comprising a Channel or Series of Channels 640, a Diversion Chamber Valve 650, a Collected Sample Valve 660, and a Bypass Flow Chamber 670. In addition, the blood collection system may include a first needle, tubing, adapter, Holder 680, a Second Needle 682, and collection bottle. As shown in FIG. 6A, before the start of the blood collection procedure, both Diversion Chamber 630 and of Bypass Flow Chamber 670 of Diversion Device 600 are empty.

The arrows in FIG. 6A indicate the direction of initial flow of blood into the Diversion Device 600. This is the path of least flow resistance without user intervention, which is driven by the vacuum pressure created by collection bottle connected to the Holder 680.

As shown in FIG. 6B, initial flow of blood fills the Channel or Series of Channels 640 of Diversion Chamber 630. Once the initial flow of blood reaches Diversion Chamber Valve 650, it prevents flow of blood through it.

As shown in FIG. 6C, subsequent flow of blood passes through Collected Sample Valve 660 into Bypass Flow Chamber 670. Bypass Flow Chamber 670 permits the subsequent flow of blood to exit Diversion Device 600 through Outlet Conduit 620 into Holder 680 and eventually to a collected bottle.

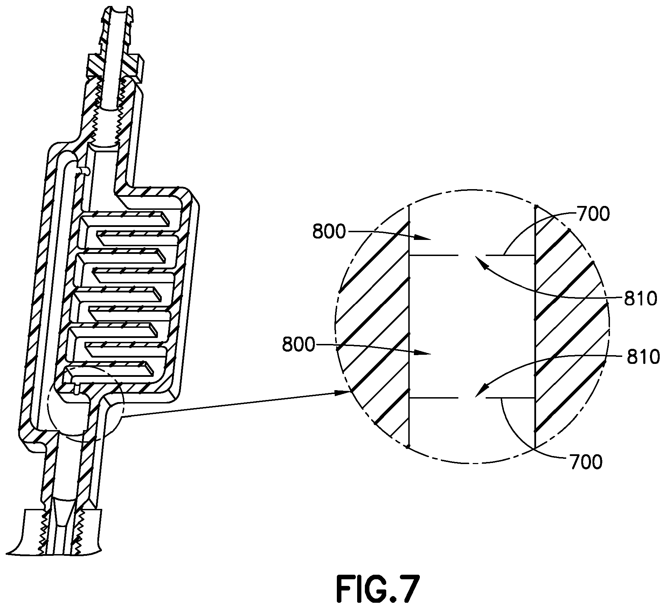

FIG. 7 illustrates an enlarged view of one embodiment of Diversion Chamber Valve 650 of the Diversion Device 600 of FIGS. 6A-6C. As shown in FIG. 7, an air gap 800 between two flow restrictors 700 ensures contaminated initial blood does not come into contact with subsequent flow of blood. As noted above, the flow restrictors 700 are made of hydrophobic material. The flow restrictors 700 also have a small orifice 810 or hole therein (e.g., about 2 mm or less) to create flow resistance therethrough. The embodiment of the Diversion Chamber Valve 650 of FIGS. 6A-6C that is illustrated in FIG. 7 has multiple air gaps and multiple flow restrictors for redundancy to ensure that no blood flows through the Diversion Chamber Valve 650 and into the Bypass Flow Chamber 670.

An air gap between flow restrictors may also be present in other embodiments of diversion chamber valve. In some embodiments, a diversion chamber valve may contain more than one set of air gap.

In some embodiments, the diversion device is a distance away from the first needle, and is close to the holder or adapter connected to a collection bottle. In some embodiments, the diversion device is close to the first needle and far away from the holder or adapter connected to a collection bottle. In a preferred example, the diversion device is part of winged butterfly first needle.

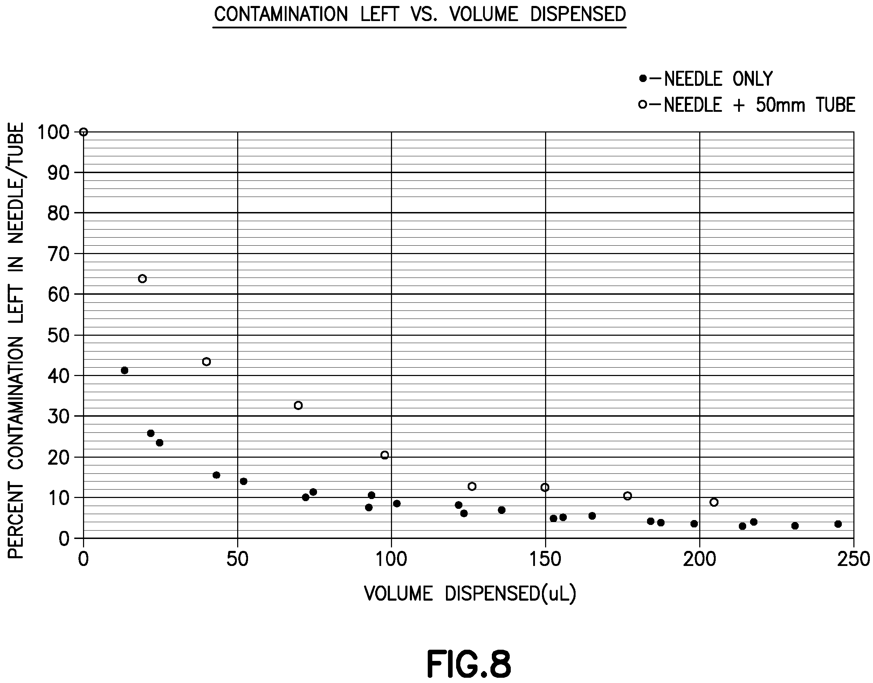

FIG. 8 shows the effect of proximity of a diversion device to a first needle. The plot in FIG. 8 shows the percent contamination that remains in a needle, and a needle with 50 mm of tubing, after a volume of clean blood flows through each. The conclusion drawn from the plot is that diversion of the contaminated blood is more likely to be effective after collecting a smaller volume of blood if the diversion device is located closer to the needle.

The volume of blood diverted into the diversion device may be changed depending on the proximity of the diversion device to the first needle. For example, in some embodiments, if the diversion device is immediately behind the first needle (e.g., as part of the winged butterfly first needle), the diversion device may be configured to direct less than about 150 µL of blood into its diversion chamber. In some embodiments, the diversion device may be configured to direct less than about 30-50 µL of blood into its diversion chamber.

As demonstrated above, some embodiments of the present invention provide significant advantages. Most organisms identified as contaminants in blood cultures originate from the skin of the patient. These contaminants are typically introduced into a patient's blood sample by the venipuncture and the initial flow of blood from the patient into a collection bottle. Therefore, by diverting and trapping an initial flow of blood, a diversion device in accordance with the present technology can potentially reduce the number of false positive blood cultures.

Furthermore, a diversion device in accordance with the present technology provides a versatile solution. For example, the distance of a diversion device to a first needle can be readily changed, so that any predetermined amount of blood, such as less than about 150 µL, can be diverted and trapped.

Moreover, the inclusion of a diversion device in accordance with the present technology in a blood collection system, does not introduce additional workflow steps for health care workers relative to presently conventional techniques for collecting blood samples. For example, health care workers do not need to wait for a conduit or a chamber to partially or completely fill before inserting a collection bottle into a holder. This advantage is achieved, in large part, because the diversion device in accordance with the present technology operate using the vacuum pressure created by a collection bottle. As a result, the diversion device in accordance with the present technology do not rely on a separate power source or the venous pressure of a patient for trapping an initial flow of blood or for collecting a subsequent flow of blood in a collection bottle.

As noted above, some embodiments of a blood collection system with a diversion device in accordance with the present technology represent closed system solutions. In these embodiments, the air that precedes the liquid blood flow is not vented out of the system and into the atmosphere. Instead, these embodiments use the vacuum pressure created by a collection bottle to immediately draw blood from a patient. A diversion device in these embodiments can be used inside the closed system to balance pressure and air flow along the flow path. For example, a diversion chamber valve can be used to allow any air preceding the blood sample to flow out of the diversion chamber and into an outlet conduit. In such embodiments, the diversion chamber valve may prevent flow therethrough of a liquid such as blood.

From the foregoing and with reference to the various figure drawings, those skilled in the art will appreciate that certain modifications can also be made to the present disclosure. For example, a diversion device in accordance with the present technology can be positioned anywhere along the flow path. For example, a diversion device in accordance with the present technology could be attached to the body of a first needle. As another example, a diversion device in accordance with the present technology could be positioned along tubing between a holder and a second needle.

Furthermore, a blood collection system in accordance with the present technology may not include all of the components illustrated in the above embodiments. For example, the needle, the diversion device, and the holder may be integrated into one device without any tubing. For example, a diversion device in accordance with the present technology could be integrated with BD's Vacutainer^{®} Eclipse^{™} blood collection needle.

Moreover, in many of the embodiments discussed above, collection bottles having a sub-atmospheric internal pressure were used to collect blood from a patient. However, a wide variety of collection vessels having a sub-atmospheric internal pressure may be used with the present technology. For example, a collection tube may be used with the present technology. As another example, a collection vial may be used with the present technology.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A diversion device (300) for collecting a biological sample, the diversion device (300) comprising:
an inlet (330) for receiving a biological sample collected from a patient;
an outlet (320) for delivering the collected biological sample to a collection vessel, the collection vessel being under sub-atmospheric pressure;
a first channel (330) into which a first portion of the collected biological sample flows upon commencement of sample collection, the first channel (330) comprising a first valve (350) such that air in the first channel (330) exits the first channel (330) through the first valve (350) as the collected biological sample fills the first channel (330);
a second channel (370) into which a second portion of the collected biological sample flows after the first channel (330) is substantially filled with collected sample, the second channel (370) in fluid communication with the first channel (330) through a second valve (360);
**characterised in that**
the outlet (320) is adapted for attachment to a needle (382) with a lumen, the needle adapted to pierce a seal on the collection vessel, such that the sub-atmospheric pressure of the collection vessel draws the biological sample from the diversion device to the collection vessel;
wherein the diversion device (300) is a closed system that prevents an initial flow of air through the diversion device from being vented into the atmosphere; and
wherein the first valve (350) is a hydrophobic flow restrictor and the second valve (360) is a hydrophobic flow restrictor, wherein the hydrophobic flow restrictor comprises a barrier that impedes a flow of liquid through an air gap in the hydrophobic flow restrictor.

2. The diversion device (300) of claim 1, wherein the inlet (330) is adapted for connection to a line set for collecting a biological sample from a patient.

3. The diversion device (300) of claim 2, wherein the line set comprises a sample collection needle (382) and a collection tube.

4. The diversion device (300) of claim 3, wherein the sample collection needle (382) is a butterfly needle selected from the group consisting of a single-winged butterfly needle or a dual wing butterfly needle.

5. The diversion device (300) of claim 4, wherein the butterfly needle is a dual winged butterfly and the diversion device (300) is integrated into one wing of the butterfly.

6. The diversion device (300) of claim 1, wherein the diversion device (300) is adapted to be coupled to an adapter that couples to a collection vessel.

7. The diversion device (300) of claim 6, wherein the adapter is coupled to the diversion device (300) by a threaded connection.

8. The diversion device (300) of claim 7, wherein the adapter is coupled to the diversion device (300) by a luer connector.

9. The diversion device (300) of claim 1, wherein the barrier in the hydrophobic flow restrictor has an orifice of about 2mm or less therein.

10. The diversion device (300) of claim 9, wherein the hydrophobic flow restrictor comprises multiple barriers with the orifice in each barrier.

11. The diversion device (300) of claim 1, wherein the first channel is a serpentine channel.

12. The diversion device (300) of any one of claims 1-11, wherein the first channel has diameter of about 3 to about 4 mm.

13. The diversion device (300) of claim 9, wherein the orifice has a diameter of about 0.5 µm or less.

14. The diversion device (300) of claim 1, wherein the hydrophobic flow restrictor is a membrane, preferably a porous membrane preferably comprising pores with a diameter of about 0.45 µm or less.

15. The diversion device (300) of any of claim 1 wherein the hydrophobic flow restrictor is made of one of polytetrafluoroethylene (PTFE) or polypropylene.

## Patentansprüche

1. Umleitungsvorrichtung (300) zur Entnahme einer biologischen Probe, wobei die Umleitungsvorrichtung (300) aufweist:
einen Einlass (330) zur Aufnahme einer von einem Patienten entnommenen biologischen Probe;
einen Auslass (320) zur Abgabe der entnommenen biologischen Probe an ein Sammelgefäß, wobei das Sammelgefäß unter subatmosphärischem Druck steht;
einen ersten Kanal (330), in den ein erster Teil der entnommenen biologischen Probe bei Beginn der Probenentnahme fließt, wobei der erste Kanal (330) ein erstes Ventil (350) aufweist, so dass Luft im ersten Kanal (330) den ersten Kanal (330) durch das erste Ventil (350) verlässt, wenn die entnommene biologische Probe den ersten Kanal (330) füllt;
einen zweiten Kanal (370), in den ein zweiter Teil der entnommenen biologischen Probe fließt, nachdem der erste Kanal (330) im Wesentlichen mit der entnommenen Probe gefüllt ist, wobei der zweite Kanal (370) durch ein zweites Ventil (360) in Fluidverbindung mit dem ersten Kanal (330) steht;
**dadurch gekennzeichnet, dass**
der Auslass (320) zur Befestigung an einer Nadel (382) mit einem Lumen geeignet ist, wobei die Nadel geeignet ist, eine Dichtung am Sammelbehälter zu durchstechen, so dass der subatmosphärische Druck des Sammelbehälters die biologische Probe aus der Umleitungsvorrichtung in den Sammelbehälter zieht;
wobei die Umleitungsvorrichtung (300) ein geschlossenes System ist, das verhindert, dass ein anfänglicher Luftstrom durch die Umleitungsvorrichtung in die Atmosphäre entlüftet wird; und
wobei das erste Ventil (350) ein hydrophober Durchflussbegrenzer ist und das zweite Ventil (360) ein hydrophober Durchflussbegrenzer ist, wobei der hydrophobe Durchflussbegrenzer eine Barriere aufweist, die einen Flüssigkeitsstrom durch einen Luftspalt in dem hydrophoben Durchflussbegrenzer verhindert.

2. Umleitungsvorrichtung (300) nach Anspruch 1, wobei der Einlass (330) für den Anschluss an einen Leitungssatz zur Entnahme einer biologischen Probe von einem Patienten geeignet ist.

3. Umleitungsvorrichtung (300) nach Anspruch 2, wobei der Leitungssatz eine Probenentnahmenadel (382) und ein Entnahmeröhrchen aufweist.

4. Umleitungsvorrichtung (300) nach Anspruch 3, wobei die Probenentnahmenadel (382) eine Butterfly-Nadel ist, die aus der Gruppe ausgewählt ist, die aus einer einflügeligen Butterfly-Nadel oder einer zweiflügeligen Butterfly-Nadel besteht.

5. Umleitungsvorrichtung (300) nach Anspruch 4, wobei die Butterfly-Nadel ein zweiflügeliger Schmetterling ist und die Umleitungsvorrichtung (300) in einen Flügel des Schmetterlings integriert ist.

6. Umleitungsvorrichtung (300) nach Anspruch 1, wobei die Umleitungsvorrichtung (300) dazu geeignet ist, mit einem Adapter gekoppelt zu werden, der mit einem Sammelbehälter gekoppelt ist.

7. Umleitungsvorrichtung (300) nach Anspruch 6, wobei der Adapter mit der Umleitungsvorrichtung (300) durch eine Gewindeverbindung gekoppelt ist.

8. Umleitungsvorrichtung (300) nach Anspruch 7, wobei der Adapter mit der Umleitungsvorrichtung (300) durch einen Luer-Anschluss gekoppelt ist.

9. Umleitungsvorrichtung (300) nach Anspruch 1, wobei die Barriere in dem hydrophoben Strömungsbegrenzer eine Öffnung von etwa 2 mm oder weniger aufweist.

10. Umleitungsvorrichtung (300) nach Anspruch 9, wobei der hydrophobe Durchflussbegrenzer mehrere Barrieren mit der Öffnung in jeder Barriere aufweist.

11. Umleitungsvorrichtung (300) nach Anspruch 1, wobei der erste Kanal ein schlangenförmiger Kanal ist.

12. Umleitungsvorrichtung (300) nach einem der Ansprüche 1-11, wobei der erste Kanal einen Durchmesser von etwa 3 bis etwa 4 mm hat.

13. Umleitungsvorrichtung (300) nach Anspruch 9, wobei die Öffnung einen Durchmesser von etwa 0,5 µm oder weniger hat.

14. Umleitungsvorrichtung (300) nach Anspruch 1, wobei der hydrophobe Strömungsbegrenzer eine Membran ist, vorzugsweise eine poröse Membran, die vorzugsweise Poren mit einem Durchmesser von etwa 0,45 µm oder weniger aufweist.

15. Umleitungsvorrichtung (300) nach Anspruch 1, wobei der hydrophobe Durchflussbegrenzer aus Polytetrafluorethylen (PTFE) oder Polypropylen besteht.

## Revendications

1. Dispositif de dérivation (300) pour prélever un échantillon biologique, le dispositif de dérivation (300) comprenant :
une entrée (330) pour recevoir un échantillon biologique prélevé chez un patient ;
une sortie (320) pour acheminer l'échantillon biologique prélevé vers un récipient de prélèvement, le récipient de prélèvement étant sous pression sub-atmosphérique ;
un premier canal (330) dans lequel s'écoule une première partie de l'échantillon biologique prélevé au début du prélèvement d'échantillon, le premier canal (330) comprenant une première valve (350) de sorte que l'air présent dans le premier canal (330) sorte du premier canal (330) par l'intermédiaire de la première valve (350) à mesure que l'échantillon biologique prélevé remplit le premier canal (330) ;
un second canal (370) dans lequel s'écoule une seconde partie de l'échantillon biologique prélevé après que le premier canal (330) est sensiblement rempli d'échantillon prélevé, le second canal (370) étant en communication fluidique avec le premier canal (330) par l'intermédiaire d'une seconde valve (360) ;
**caractérisé en ce que**
la sortie (320) est adaptée pour être fixée à une aiguille (382) avec une lumière, l'aiguille étant adaptée pour percer un joint sur le récipient de prélèvement, de sorte que la pression sub-atmosphérique du récipient de prélèvement aspire l'échantillon biologique du dispositif de dérivation vers le récipient de prélèvement ;
dans lequel le dispositif de dérivation (300) est un système fermé qui empêche un écoulement initial d'air à travers le dispositif de dérivation d'être évacué dans l'atmosphère ; et
dans lequel la première valve (350) est un limiteur d'écoulement hydrophobe et la seconde valve (360) est un limiteur d'écoulement hydrophobe, dans lequel le limiteur d'écoulement hydrophobe comprend une barrière qui empêche un écoulement de liquide à travers un espace d'air dans le limiteur d'écoulement hydrophobe.

2. Dispositif de dérivation (300) selon la revendication 1, dans lequel l'entrée (330) est adaptée pour être reliée à un ensemble de lignes pour prélever un échantillon biologique chez un patient.

3. Dispositif de dérivation (300) selon la revendication 2, dans lequel l'ensemble de lignes comprend une aiguille de prélèvement d'échantillon (382) et un tube de prélèvement.

4. Dispositif de dérivation (300) selon la revendication 3, dans lequel l'aiguille de prélèvement d'échantillon (382) est une aiguille à ailettes choisie dans le groupe constitué par une aiguille à ailettes simple ou une aiguille à ailettes double.

5. Dispositif de dérivation (300) selon la revendication 4, dans lequel l'aiguille à ailettes est une aiguille à ailettes double et le dispositif de dérivation (300) est intégré dans une ailette de l'aiguille à ailettes.

6. Dispositif de dérivation (300) selon la revendication 1, dans lequel le dispositif de dérivation (300) est adapté pour être couplé à un adaptateur qui se couple à un récipient de prélèvement.

7. Dispositif de dérivation (300) selon la revendication 6, dans lequel l'adaptateur est couplé au dispositif de dérivation (300) par un raccordement fileté.

8. Dispositif de dérivation (300) selon la revendication 7, dans lequel l'adaptateur est couplé au dispositif de dérivation (300) par un raccord Luer.

9. Dispositif de dérivation (300) selon la revendication 1, dans lequel la barrière dans le limiteur d'écoulement hydrophobe présente, à l'intérieur, un orifice d'environ 2 mm ou moins.

10. Dispositif de dérivation (300) selon la revendication 9, dans lequel le limiteur d'écoulement hydrophobe comprend de multiples barrières avec l'orifice dans chaque barrière.

11. Dispositif de dérivation (300) selon la revendication 1, dans lequel le premier canal est un canal en serpentin.

12. Dispositif de dérivation (300) selon l'une quelconque des revendications 1 à 11, dans lequel le premier canal a un diamètre d'environ 3 à environ 4 mm.

13. Dispositif de dérivation (300) selon la revendication 9, dans lequel l'orifice a un diamètre inférieur ou égal à environ 0,5 µm.

14. Dispositif de dérivation (300) selon la revendication 1, dans lequel le limiteur d'écoulement hydrophobe est une membrane, de préférence une membrane poreuse comprenant de préférence des pores d'un diamètre inférieur ou égal à environ 0,45 µm.

15. Dispositif de dérivation (300) selon la revendication 1, dans lequel le limiteur d'écoulement hydrophobe est constitué de l'un parmi le polytétrafluoroéthylène (PTFE) ou le polypropylène.
